# EUROPEAN PATENT SPECIFICATION

(11) **EP 4 542 485 B1**
(45) Date of publication and mention of the grant of the patent: **25.02.2026**
(21) Application number: 24203051.8
(22) Date of filing: 26.09.2024
(51) Int. Cl.: G06T 7/00, G06T 7/11, G06V 20/69

(54) **SYSTEMS AND METHODS FOR DETECTING LUNG POINT**
SYSTEME UND VERFAHREN ZUR ERKENNUNG DES LUNGENPUNKTES
SYSTÈMES ET PROCÉDÉS DE DÉTECTION DE POINT PULMONAIRE

(30) Priority: 26.09.2023 US 202363585391 P
(43) Date of publication of application: 23.04.2025
(73) Proprietor: Deep Breathe Inc., London, ON N6A 1B8 (CA)
(72) Inventor: ARNTFIELD, Robert Thomas, London, N6C 2G5 (CA); VANBERLO, Blake, Lucan, N0M 2J0 (CA); SMITH, Delaney, Port Perry, L9L 1B4 (CA); RAHMAN, Marwan, Mississauga, L5B 1J9 (CA); ROSHANKAR, Amir, London, N6A 5P2 (CA)
(74) Representative: Marks & Clerk LLP

(56) References cited:
- JASCUR MIROSLAV ET AL: "Detecting the Absence of Lung Sliding in Lung Ultrasounds Using Deep Learning", APPLIED SCIENCES, vol. 11, no. 15, 29 July 2021 (2021-07-29), pages 6976, XP093254048, ISSN: 2076-3417, DOI: 10.3390/app11156976
- VANBERLO BLAKE ET AL: "Accurate assessment of the lung sliding artefact on lung ultrasonography using a deep learning approach", COMPUTERS IN BIOLOGY AND MEDICINE, NEW YORK, NY, US, vol. 148, 9 August 2022 (2022-08-09), XP087161867, ISSN: 0010-4825, [retrieved on 20220809], DOI: 10.1016/J.COMPBIOMED.2022.105953

## Description

### CROSS-REFERENCE TO RELATED APPLICATION

This present application claims priority to United States Provisional Patent Application No. 63/585,391, filed on September 26, 2023, and titled "SYSTEMS AND METHODS FOR DETECTING LUNG POINT".

### TECHNICAL FIELD

The disclosed exemplary embodiments relate to automated processing and, in particular, to automated processing of medical images and videos.

### BACKGROUND

Lung sliding is a feature used in lung ultrasound to assess lung health. The presence of lung sliding indicates normal lung movement and ventilation. However, the absence of lung sliding is suggestive of lung pathology, such as pneumothorax, that can be life-threatening. In some cases, clinicians use lung ultrasound as a valuable tool for rapid assessments at the bedside, especially in emergency situations where a quick evaluation of lung function is crucial.

Lung sliding refers to the dynamic interaction between the two pleural layers of the lung, in some cases, the visceral pleura and the parietal pleura. The presence of lung sliding signifies the juxtaposition of the two pleural layers that move synchronously during breathing. In some cases, employing a high frequency linear transducer enhances the visualization of this sign. In some cases, a pleural line is a hyperechoic line that is visible in lung ultrasound imagery that represents the junction of the visceral and the parietal pleura.

### SUMMARY

The following summary is intended to introduce the reader to various aspects of the detailed description, but not to define or delimit any invention.

In at least one broad aspect, a computing system is provided for processing medical imagery of a lung, comprising a memory storing instructions; and a processor coupled to the memory. The processor is configured to execute the instructions to:
automatically process a plurality of B-mode video frames from a video clip of the lung to generate a plurality of M-mode images associated with the ultrasound video clip;
process the plurality of M-mode images using an image classifier to output a plurality of confidence values respectively corresponding to the plurality of M-mode images; and
process the plurality of confidence values (e.g., also called prediction confidences or M-mode prediction confidences) using a clip prediction module to output a binary class prediction, which indicates lung sliding is present or absent in the video clip.

In some cases, the processor is further configured to add a bounding box to the plurality of B-mode video frames, wherein the bounding box encompasses a pleural line, and the plurality of M-mode images intersect the bounding box.

In some cases, the processor is further configured to execute a machine learning model to image process one or more of the plurality of B-mode video frames to compute a location of the bounding box that encompasses the pleural line.

In some cases, the processor is further configured to execute a process to determine a location of the bounding box, the process comprising:
computing a video clip average of pixel intensities across a time dimension of the plurality of B-mode video frames;
rescaling all pixel intensities across the plurality of B-mode video frames using the video clip average, with rescaled pixel intensities in a range [0, 1];
increasing an image contrast in each of the plurality of B-mode video frames;
applying a Radon Transform to rotate the plurality of B-mode video frames;
applying a thresholding process to extract a region of interest in the plurality of B-mode video frames;
applying horizontal erosion and horizontal dilation to the plurality of B-mode video frames;
applying a contour finding process to identify a plurality of contours that potentially bound the pleural line;
identify a brightest contour from amongst the plurality of contours that comprises a sum of pixel intensities that is greatest, wherein the sum of pixel intensities are associated with coordinates which are below and within x-coordinate bounds of the brightest contour; and
compute the bounding box around the brightest contour.

In some cases, the processor is configured to divide the video clip into a plurality of clip segments; and
for each one of the plurality of clip segments, the processor is configured to:
   a. perform binning for each clip segment, to divide x-coordinates of the pleural line into contiguous chunks with equal width;
   b. obtain a predicted class for each clip segment; and
when at least one of the clip segments has the predicted class that indicates an absence of lung sliding, output a prediction for the video clip indicating the absence of lung sliding.

In some cases, prior to obtaining the predicted class for each one of the plurality of clip segments, the processor is further configured to compute a moving average of the M-mode prediction confidences for each clip segment.

In some cases, the processor is configured to divide the video clip into a plurality of clip segments; and
for each one of the plurality of clip segments, the processor is configured to:
   a. perform binning for each clip segment, to divide x-coordinates of the pleural line into contiguous chunks with equal width, resulting in a plurality of bins;
   b. identify a brightest M-mode image in each of the plurality of bins;
   c. apply a classification thresholding process to the prediction confidence for each one of the brightest M-mode images to obtain a class prediction for each of the plurality of bins;
   d. when at least one of the class predictions indicates an absence of lung sliding, set a prediction of the clip segment to indicate the absence of lung sliding; and
when at least one of the plurality of clip segments has the prediction indicating the absence of lung sliding, then output a prediction for the video clip indicating the absence of lung sliding.

In some cases, the processor is configured to divide the video clip into a plurality of clip segments; and
for each one of the plurality of clip segments, the processor is configured to:
   a. perform binning for each clip segment, to divide x-coordinates of the pleural line into contiguous chunks with equal width, resulting in a plurality of bins;
   b. for each of the plurality of bins, determine a mean prediction confidence for its constituent M-mode images;
   c. apply a classification thresholding process to an averaged prediction confidence to compute a class prediction for each of the plurality of bins;
   e. when at least one of the class predictions indicates an absence of lung sliding, set a prediction of the clip segment indicating the absence of lung sliding; and
when at least one of the plurality of clip segments has the prediction indicating the absence of lung sliding, then output a prediction for the video clip indicating the absence of lung sliding.

In some cases, the processor is configured to divide the video clip into a plurality of clip segments; and
for each one of the plurality of clip segments, the processor is configured to:
   a. perform binning for each clip segment, to divide x-coordinates of the pleural line into contiguous chunks with equal width, resulting in a plurality of bins;
   b. replace a list of prediction confidences for a given clip segment with its moving average;
   c. compute a moving average of a brightness of each M-mode image at each x-coordinate of the pleural line;
   d. identify a M-mode image in each of the plurality of bins with the greatest brightness moving average;
   e. apply a classification thresholding process to a prediction confidence for each identified M-mode image to compute a class prediction for each of the plurality of bins;
   f. when at least one of the class predictions indicates an absence of lung sliding, set a prediction of the clip segment indicating the absence of lung sliding; and
when at least one of the plurality of clip segments has the prediction indicating the absence of lung sliding, then output a prediction for the video clip indicating the absence of lung sliding.

In some cases, the processor is configured to divide the video clip into a plurality of clip segments; and
for each one of the plurality of clip segments, the processor is configured to:
   a. perform binning for each clip segment, to divide x-coordinates of the pleural line into contiguous chunks with equal width, resulting in a plurality of bins;
   b. replace a list of prediction confidences for a given clip segment with its moving average;
   c. identify a M-mode image corresponding to a midpoint of each bin from the plurality of bins;
   d. apply a classification thresholding process to a prediction confidence for each identified M-mode image to compute a class prediction for each of the plurality of bins;
   e. when at least one of the class predictions indicates an absence of lung sliding, set a prediction of the clip segment indicating the absence of lung sliding; and
when at least one of the plurality of clip segments has the prediction indicating the absence of lung sliding, then output a prediction for the video clip indicating the absence of lung sliding.

In some cases, the processor is configured to divide the video clip into a plurality of clip segments; and
for each one of the plurality of clip segments, the processor is configured to:
   a. perform binning for each clip segment, to divide x-coordinates of the pleural line into contiguous chunks with equal width, resulting in a plurality of bins;
   b. identify a M-mode image corresponding to a midpoint of a range of prediction confidences for each bin from amongst the plurality of bins;
   c. apply a classification thresholding process to a prediction confidence for each identified M-mode image to compute a class prediction for each of the plurality of bins;
   d. when at least one of the class predictions indicates an absence of lung sliding, set a prediction of the clip segment indicating the absence of lung sliding; and
when at least one of the plurality of clip segments has the prediction indicating the absence of lung sliding, then output a prediction for the video clip indicating the absence of lung sliding.

In some cases, the processor is configured to divide the video clip into a plurality of clip segments; and
for each one of the plurality of clip segments, the processor is configured to:
   a. perform binning for each clip segment, to divide x-coordinates of the pleural line into contiguous chunks with equal width, resulting in a plurality of bins;
   b. for each one of the plurality of bins, identify a M-mode image corresponding to a median of prediction confidences for that bin;
   c. apply a classification thresholding process to a prediction confidence for each identified M-mode image to compute a class prediction for each of the plurality of bins;
   d. when at least one of the class predictions indicates an absence of lung sliding, set a prediction of the clip segment indicating the absence of lung sliding; and
when at least one of the plurality of clip segments has the prediction indicating the absence of lung sliding, then output a prediction for the video clip indicating the absence of lung sliding.

In another broad aspect, a method is provided for processing medical imagery of a lung, the method executed in a computing environment comprising one or more processors and memory. The method comprises:
automatically processing a plurality of B-mode video frames from a video clip of the lung to generate a plurality of M-mode images associated with the ultrasound video clip;
processing the plurality of M-mode images using an image classifier to output a plurality of confidence values (e.g., also called prediction confidences or M-mode prediction confidences) respectively corresponding to the plurality of M-mode images; and
processing the plurality of confidence values using a clip prediction module to output a binary class prediction, which indicates lung sliding is present or absent in the video clip.

In some cases, the method further comprises adding a bounding box to the plurality of B-mode video frames, wherein the bounding box encompasses a pleural line, and the plurality of M-mode images intersect the bounding box.

In some cases, the method further comprises executing a machine learning model to image process one or more of the plurality of B-mode video frames to compute a location of the bounding box that encompasses the pleural line.

In some cases, the method further comprises executing a process to determine a location of the bounding box, the process comprising:
computing a video clip average of pixel intensities across a time dimension of the plurality of B-mode video frames;
rescaling all pixel intensities across the plurality of B-mode video frames using the video clip average, with rescaled pixel intensities in a range [0, 1];
increasing an image contrast in each of the plurality of B-mode video frames;
applying a Radon Transform to rotate the plurality of B-mode video frames;
applying a thresholding process to extract a region of interest in the plurality of B-mode video frames;
applying horizontal erosion and horizontal dilation to the plurality of B-mode video frames;
applying a contour finding process to identify a plurality of contours that potentially bound the pleural line;
identifying a brightest contour from amongst the plurality of contours that comprises a sum of pixel intensities that is greatest, wherein the sum of pixel intensities are associated with coordinates which are below and within x-coordinate bounds of the brightest contour; and
computing the bounding box around the brightest contour.

In some cases, the method further comprises dividing the video clip into a plurality of clip segments; and
for each one of the plurality of clip segments:
   a. performing binning for each clip segment, to divide x-coordinates of the pleural line into contiguous chunks with equal width;
   b. obtaining a predicted class for each clip segment; and
when at least one of the clip segments has the predicted class indicating an absence of lung sliding, outputting a prediction for the video clip indicating the absence of lung sliding.

In some cases, the method further comprises, prior to obtaining the predicted class for each clip segment, computing a moving average of the M-mode prediction confidences for each clip segment.

In some cases, the method further comprises dividing the video clip into a plurality of clip segments; and
for each one of the plurality of clip segments:
   a. performing binning for each clip segment, to divide x-coordinates of the pleural line into contiguous chunks with equal width, resulting in a plurality of bins;
   b. identifying a brightest M-mode image in each of the plurality of bins;
   c. applying a classification thresholding process to the prediction confidence for each one of the brightest M-mode images to obtain a class prediction for each of the plurality of bins;
   d. when at least one of the class predictions indicates an absence of lung sliding, setting a prediction of the clip segment indicating the absence of lung sliding; and
when at least one of the plurality of clip segments has the prediction indicating the absence of lung sliding, then outputting a prediction for the video clip indicating the absence of lung sliding.

In some cases, the method further comprises dividing the video clip into a plurality of clip segments; and
for each one of the plurality of clip segments:
   a. performing binning for each clip segment, to divide x-coordinates of the pleural line into contiguous chunks with equal width, resulting in a plurality of bins;
   b. for each of the plurality of bins, determining a mean prediction confidence for its constituent M-mode images;
   c. applying a classification thresholding process to an averaged prediction confidence to compute a class prediction for each of the plurality of bins;
   d. when at least one of the class predictions indicates an absence of lung sliding, setting a prediction of the clip segment indicating the absence of lung sliding; and
when at least one of the plurality of clip segments has the indicating the absence of lung sliding, then outputting a prediction for the video clip indicating the absence of lung sliding.

In some cases, the method further comprises dividing the video clip into a plurality of clip segments; and
for each one of the plurality of clip segments:
   a. performing binning for each clip segment, to divide x-coordinates of the pleural line into contiguous chunks with equal width, resulting in a plurality of bins;
   b. replacing a list of prediction confidences for a given clip segment with its moving average;
   c. computing a moving average of a brightness of each M-mode image at each x-coordinate of the pleural line;
   d. identifying a M-mode image in each of the plurality of bins with the greatest brightness moving average;
   e. applying a classification thresholding process to a prediction confidence for each identified M-mode image to compute a class prediction for each of the plurality of bins;
   f. when at least one of the class predictions indicate an absence of lung sliding, setting a prediction of the clip segment indicating the absence of lung sliding; and
when at least one of the plurality of clip segments has the prediction indicating the absence of lung sliding, then output a prediction for the video clip indicating the absence of lung sliding.

In some cases, the method further comprises dividing the video clip into a plurality of clip segments; and
for each one of the plurality of clip segments:
   a. performing binning for each clip segment, to divide x-coordinates of the pleural line into contiguous chunks with equal width, resulting in a plurality of bins;
   b. replacing a list of prediction confidences for a given clip segment with its moving average;
   c. identifying a M-mode image corresponding to a midpoint of each bin from the plurality of bins;
   d. applying a classification thresholding process to a prediction confidence for each identified M-mode image to compute a class prediction for each of the plurality of bins;
   e. when at least one of the class predictions indicate an absence of lung sliding, setting a prediction of the clip segment indicating the absence of lung sliding; and
when at least one of the plurality of clip segments has the prediction indicating the absence of lung sliding, then outputting a prediction for the video clip indicating the absence of lung sliding.

In some cases, the method further comprises dividing the video clip into a plurality of clip segments; and
for each one of the plurality of clip segments:
   a. performing binning for each clip segment, to divide x-coordinates of the pleural line into contiguous chunks with equal width, resulting in a plurality of bins;
   b. identifying a M-mode image corresponding to a midpoint of a range of prediction confidences for each bin from amongst the plurality of bins;
   c. applying a classification thresholding process to a prediction confidence for each identified M-mode image to compute a class prediction for each of the plurality of bins;
   d. when at least one of the class predictions indicates an absence of lung sliding, setting a prediction of the clip segment indicating the absence of lung sliding; and
when at least one of the plurality of clip segments has the indicating the absence of lung sliding, then output a prediction for the video clip indicating the absence of lung sliding.

In some cases, the method further comprises dividing the video clip into a plurality of clip segments; and
for each one of the plurality of clip segments, the processor is configured to:
a. performing binning for each clip segment, to divide x-coordinates of the pleural line into contiguous chunks with equal width, resulting in a plurality of bins;
b. for each one of the plurality of bins, identifying a M-mode image corresponding to a median of prediction confidences for that bin;
c. applying a classification thresholding process to a prediction confidence for each identified M-mode image to compute a class prediction for each of the plurality of bins;
d. when at least one of the class predictions indicates an absence of lung sliding, setting a prediction of the clip segment indicating the absence of lung sliding; and
when at least one of the plurality of clip segments has the prediction indicating the absence of lung sliding, then output a prediction for the video clip indicating the absence of lung sliding.

According to some aspects, the present disclosure provides a non-transitory computer-readable medium storing computer-executable instructions. The computer-executable instructions, when executed, configure a processor to perform any of the methods described herein.

### BRIEF DESCRIPTION OF THE DRAWINGS

The drawings included herewith are for illustrating various examples of articles, methods, and systems of the present specification and are not intended to limit the scope of what is taught in any way. In the drawings:
FIG. 1 is an M-mode image showing a vertical slice through time taken from a B-mode video;
FIG. 2 is a schematic block diagram of a computing system configured to process medical imagery of a lung and predict if lung sliding is present or absent;
FIG. 3 is a block diagram of a computer in accordance with at least some embodiments;
FIGS. 4A to 4F provide a schematic representation of the methods underlying each 3-second clip segment prediction;
FIGS. 5A to 5C provide a comparison of the lung sliding (FIG. 5A), absent lung sliding (FIG. 5B), and lung point (FIG. 5C) artifacts on B-Mode (i and ii) and M-Mode (iii and iv) ultrasound;
FIG. 6 is a still frame from a B-mode video with multiple pleural line fragments;
FIG. 7 is a still frame from a B-mode video where the pleural line ROI has been split into b=4 bins of equal width;
FIG. 8 is a visual representation of a method in accordance with at least some embodiments; and
FIG. 9 is a flow diagram of executable instructions for processing medical imagery of a lung.

### DETAILED DESCRIPTION

The described systems and methods generally take a B-mode lung ultrasound video as input and return a binary prediction for whether there is evidence of absent lung sliding. In some cases, B-mode refers brightness mode. The use of grey scale imaging in ultrasound renders a two-dimensional image in which the organs and tissues of interest are depicted as points of variable brightness. The formation of a B-mode image relies on the pulse-echo principle; assuming the speed of sound remains constant, the position of a target of interest may be inferred by the time taken from emission to its return to the transducer. In some cases, to construct a cross-sectional image, the pulse-echo sequences from a multitude of neighboring scan lines are sequentially summated in real-time, generating a moving image. The two-dimensional ultrasound image includes bright dots representing the ultrasound echoes. The brightness of each dot is determined by the amplitude of the returned echo signal. This allows for visualization and quantification of anatomical structures, as well as for the visualization of diagnostic and therapeutic procedures.

Generally, if absent lung sliding is present anywhere throughout the video in the spatial or temporal dimensions, the system's decision is "Absent Lung Sliding" (even if some regions show evidence of lung sliding). Generally, if lung sliding is present everywhere throughout the video, the system's decision is "Lung Sliding", meaning that the practitioner can rule out a diagnosis of pneumothorax (PTX) at the site of the ultrasound probe.

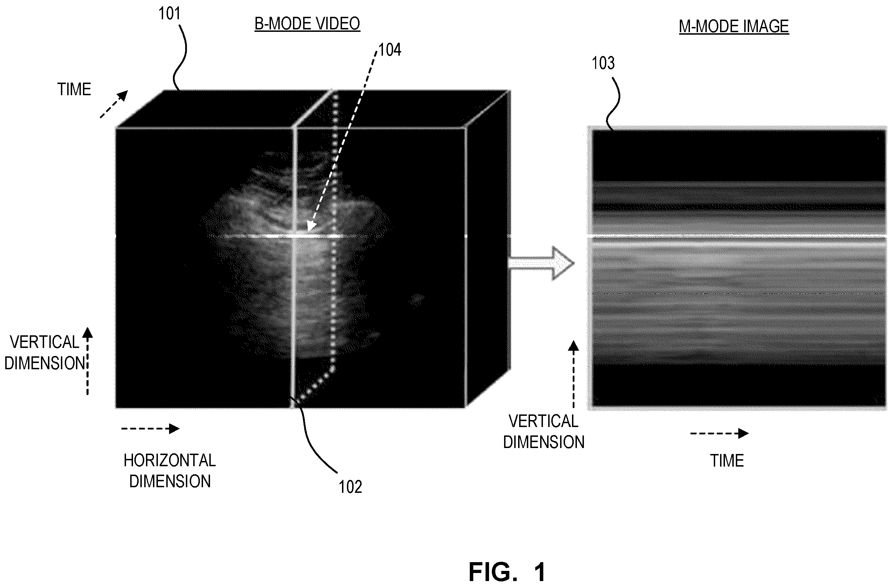

The described embodiments make use of M-mode ultrasound images, which can be described as a vertical slice of a B-mode video through time. As illustrated in FIG. 1, B-mode video 101 is illustrated as a series of video frames each having vertical dimension and a horizontal dimension. The video frames may vary over time. A vertical slice 102 through each of the B-mode video frames is used to form the M-mode image 103. The horizontal dimension of an M-mode image is time, and the vertical dimension is the vertical dimension of the video. In some cases, M-mode imaging refers to motion mode imaging. M-mode imaging includes axial and temporal resolution of structures, in which a single scan line may be emitted, received, and displayed graphically. In some cases, the B-mode video 101 captures a pleural line 104. In some cases, the computing system and method described herein generates one or more M-mode images that intersect the pleural line 104 and processes the same to determine that lung sliding is present or absent.

In some cases, a system and a method are provided that include a deep convolutional neural network that predicts whether M-mode images contain evidence for lung sliding or absent lung sliding. M-modes are eligible for consideration if they intersect the pleural line artifact.

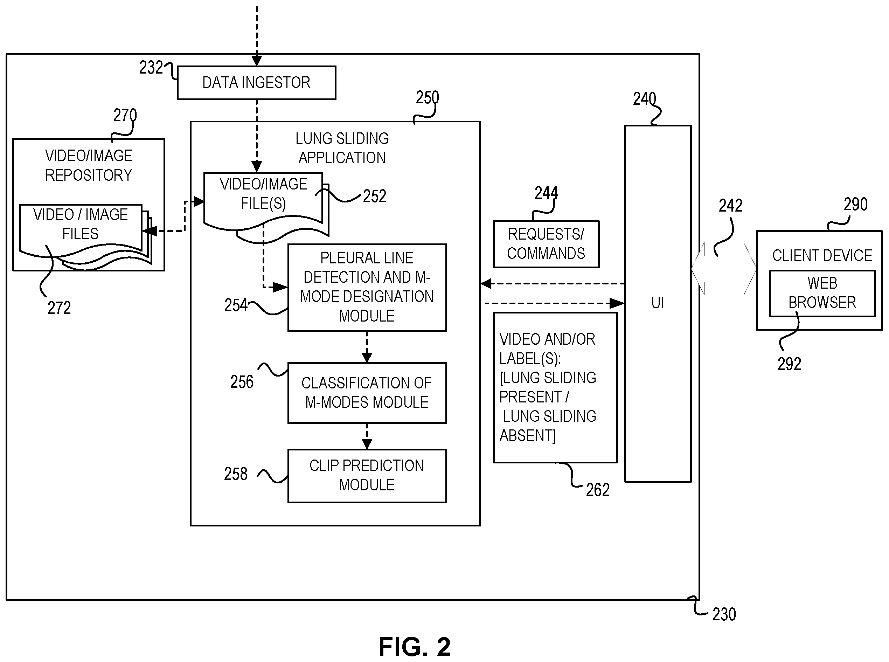

Referring now to FIG. 2, an example of a computing system 230 is shown that is configured to execute computations to process B-mode video and automatically determine if lung sliding is present or absent. The computing system 230 includes a lung sliding application 250, a user interface 240, and a video and/or image repository 270 that stores thereon ultrasound video files or image files, or both 272. In some cases, the computing system 230 includes a data ingestor 232 that obtains ultrasound video and/or image files 252, which may be processed directly by the lung sliding application 250 or may be stored in the video and/or image repository 270. In other words, in some cases, ultrasound video files or ultrasound images, or both, could be obtained by using the data ingestor 232 or could be obtained from the video and/or image repository 270.

In some cases, the ultrasound video files or ultrasound images files, or both, 252 are images of a lung, which are inputted into the lung sliding application 250. The lung sliding application 250 processes the video and/or images and determines if lung sliding is present or absent in the video and/or images.

In some cases, an output 262 from the lung sliding application includes a classification or a label of the video file or image file, which indicates whether lung sliding is present, or lung sliding is absent. In some cases, a confidence value associated with the classification or label is provided in the output 262. In some cases, the processed and analyzed ultrasound video files or ultrasound image files are included in the output 262. In some cases, the output 262 is sent to a user interface (UI) module 240 that is displayable on a client device 290, such as a web browser 292 that operates on the client device 290.

In some cases, the client device 290 sends requests or commands 244 to the UI module 240 via a communication link 242 (e.g., a network link) and the requests or commands 244 are transmitted to the ultrasound lung sliding application 250. In some cases, the requests or commands 244 include initiating processing of selected ultrasound video files or selected ultrasound images files, or both, that include lung imagery.

In some cases, the described systems and methods are split into three broad modules: a pleural line detection and M-mode designation module 254; a classification of M-modes module 256; and a clip prediction module 256. These modules are part of the lung sliding application 250.

Pleural line detection and M-mode designation module 254: In some cases, this module outputs a bounding box that contains the pleural line artifact throughout the duration of the video. The box can be described as the location of the top left corner, along with its width and height. In some cases, M-mode images that intersect the pleural line bounding boxes are eligible for classifier prediction.

Classification of M-modes module 256: In some cases, this module 256 includes a convolutional neural network binary classifier. Each M-mode image is passed through this convolutional neural network binary classifier, which predicts lung sliding (negative class) or absent lung sliding (positive class). This module outputs confidence p in a range [0, 1]. The predicted class is negative if p is less than classification threshold t, or positive otherwise.

Clip Prediction Module 258: This module 258 translates the series of constituent M-mode-level prediction confidences for each B-mode into a binary prediction for the entire clip, indicating whether the clip contains evidence of absent lung sliding.

The computing system 230 is an example embodiment. In some cases, the computing system 230 is a server machine. In some other cases, the computing system 230 is a desktop computer. In some other cases, the computing system 230 is a virtual machine instantiated as a processing node on a cloud computing platform. Other computing architectures for computing ultrasound masking could be used.

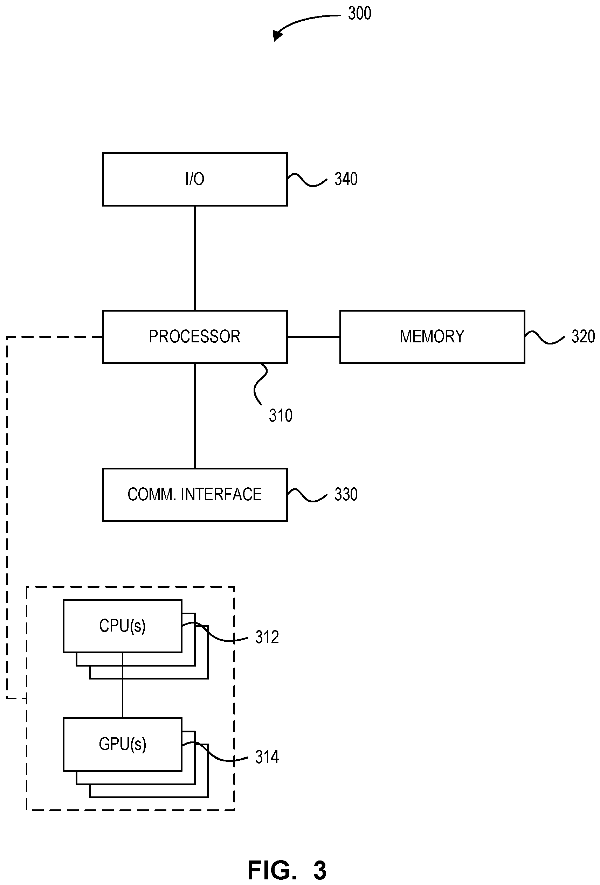

Referring now to FIG. 3, there is illustrated a simplified block diagram of a computer in accordance with at least some embodiments. Computer 300 is an example implementation of the computing system 230 or client device 290, or both, of FIG. 2. Computer 300 has at least one processor 310 operatively coupled to at least one memory 320, at least one communications interface 330 (also herein called a network interface), and at least one input/output device 340.

The at least one memory 320 includes a volatile memory that stores instructions executed or executable by processor 310, and input and output data used or generated during execution of the instructions. Memory 320 may also include non-volatile memory used to store input and/or output data - e.g., within a database - along with program code containing executable instructions.

Processor 310 may transmit or receive data via communications interface 330, and may also transmit or receive data via any additional input/output device 340 as appropriate.

In some cases, the processor 310 includes a system of central processing units (CPUs) 312. In some other cases, the processor includes a system of one or more CPUs and one or more Graphical Processing Units (GPUs) 314 that are coupled together. For example, the lung sliding application 250 executes machine learning computations, including convolutional neural network classifier computations, on CPU and GPU hardware, such as the system of CPUs 312 and GPUs 314.

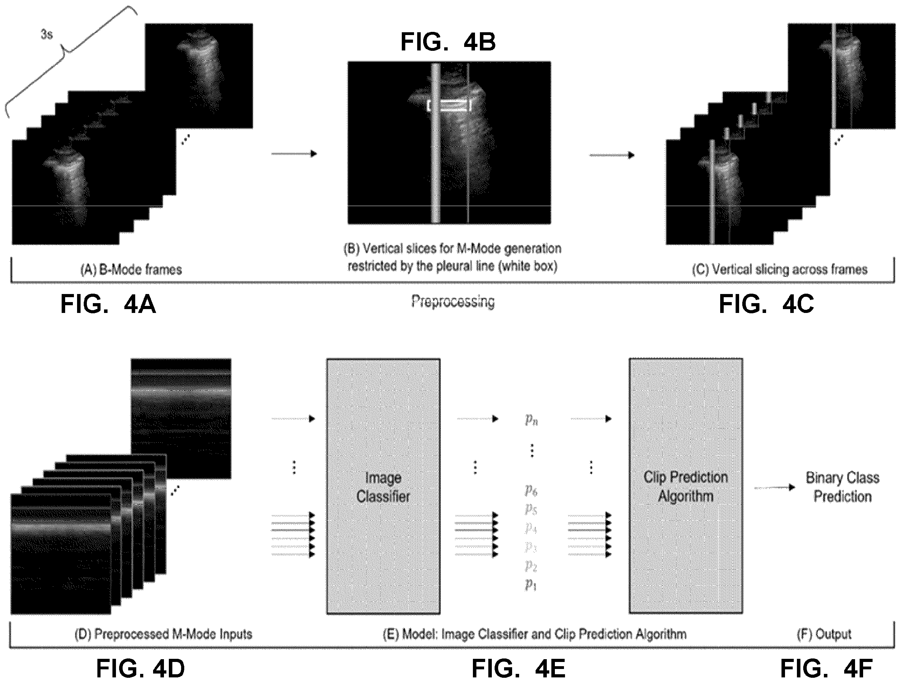

An overview of the methodology is illustrated in FIGs. 4A to 4F, which provide a schematic representation of the methods underlying each 3-second clip segment prediction. Referring to FIGs. 4A to 4D, for each masked 3-second B-Mode video clip, a set of *n* M-Mode images are generated during preprocessing by slicing through the pleural line at *n* different positions through time. *n* is counting number. In particular, FIG. 4A shows a set of B-mode frames (in a time series) that form the 3-second video clip. FIG. 4B shows *n* vertical slices demarcated in a given B-mode frames that are used for M-mode image generation. In some cases, the vertical slices for M-mode image generation are bound to a region of interest, such as the pleural line shown by the box in FIG. 4B. FIG. 4C shows vertical slicing across each frame in the set of B-mode frames. In some cases, the operations and data components shown in FIGs. 4A to 4D are executed by the pleural line detection and M-mode designation module 254.

In FIG. 4E, these *n* inputs are sent to the model, which consists of an image classifier and a clip prediction algorithm. The image classifier predicts the confidence p of absent lung sliding at the M-Mode-level. The latter converts the resulting series of n M-Mode-level predictions (e.g., p₁, p₂, p₃, ..., pₙ), also called confidence values or prediction confidences or M-mode prediction confidences, into a single binary prediction (shown in FIG. 4F) of "Lung Sliding Absent" or "Lung Sliding Present" for the clip segment. In some cases, the image classifier shown in FIG. 4E is part of the classification of M-modes module 256. In some cases, the image classifier is a convolutional neural network binary classifier. In some cases, the clip prediction algorithm in FIG. 4E is part of the clip prediction module 258.

The use of a neural network for detecting absent lung sliding in humans based on M-mode lung ultrasound images has been suggested by Jaščur et al., "Detecting the Absence of Lung Sliding in Lung Ultrasounds Using Deep Learning," Appl. Sci. 2021, 11, 6976, https://doi.org/10.3390/app11156976 and VanBerlo et al., "Accurate assessment of the lung sliding artefact on lung ultrasonography using a deep learning approach," Comput Biol Med. 2022 Sep;148:105953, doi: 10.1016/j.compbiomed.2022.105953, Epub 2022 Aug 9, PMID: 35985186.

In some cases, the described embodiments provided herein improve on prior approaches in the following areas:
- Pleural line detection - multiple strategies could be used for identifying the pleural line. The described embodiments employ two approaches to this: (1) a machine learning object detection approach and (2) a novel explicit algorithmic approach that relies on computer vision methods.
- Clip classification methods - an important special case of the positive class occurs in lung point scenarios. In lung point, an artifact that is definitive of PTX, part of the pleural line exhibits lung sliding, and the other part exhibits absent lung sliding. The most clinically significant finding is the existence of absent lung sliding; therefore, the Clip Prediction Algorithm would output "Absent Lung Sliding" (or some other indication of an absence of lung sliding) under these circumstances. The described embodiments provide multiple approaches for distilling multiple M-mode-level confidence levels into a single binary decision for whether a B-mode clip contains evidence of absent lung sliding.

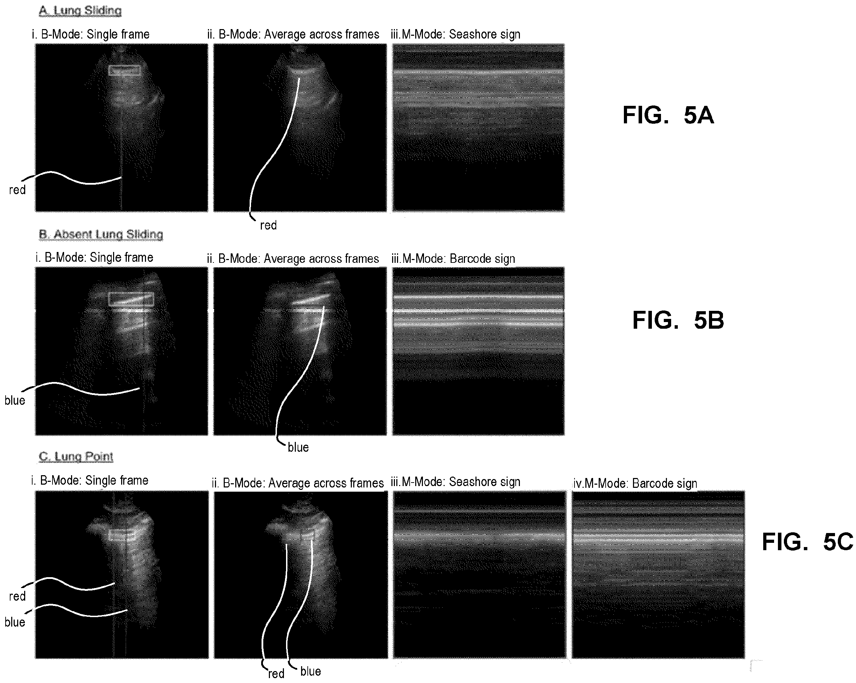

FIGs. 5A to 5C provide a clear visual representation of the lung point scenario and how it compares to the more common scenarios where there is only evidence of either present or absent lung sliding.

In FIGs. 5A to 5C, there is shown a comparison of the lung sliding (FIG. 5A), absent lung sliding (FIG. 5B), and lung point (FIG. 5C) artifacts on B-Mode (panels i and ii) and M-Mode (panels iii and iv) ultrasound. The color red in the vertical lines and/or bounding boxes signifies the presence of lung sliding. The color blue in the vertical lines and/or bounding boxes signifies the absence of lung sliding. Bounding boxes highlight the location of the pleural line on single and averaged B-Mode frames in panels i and ii, respectively. Vertical lines indicate the B-Mode slices used to produce the M-Mode images displayed in panels iii and iv. In FIG. 5A: The lung sliding artifact is present across the entirety of the pleural line. M-Modes sliced through any horizontal index intersecting the pleural line will display the seashore sign, for example as described in Lichtenstein, "Whole Body Ultrasonography in the Critically III," Springer Science & Business Media

(2010), doi: https://doi.org/10.1007/978-3-642-05328-3. In FIG. 5B: The lung sliding artifact is absent across the entirety of the pleural line. M-Modes sliced through any horizontal index intersecting the pleural line will display the barcode sign. In FIG. 5C: A transition from a sliding to a static pleural line is visualized (i.e., the lung sliding artifact is both present and absent in the same B-Mode). M-Modes sliced at indices to the left of the lung point will display the seashore sign. M-Modes sliced at indices to the right of the lung point will display the barcode sign.

### Pleural line detection & M-mode designation

In this section of the described approaches, eligible M-mode images are extracted from an input B-mode ultrasound video. FIGS. 4A to 4D summarize the product of this step. Eligible M-mode images include those that intersect the pleural line. It is therefore helpful to identify the horizontal bounds of the pleural line. The described embodiments provide two methods for determining the location of the pleural line. Both methods output possible x-coordinates that intersect the pleural line, permitting M-mode extraction.

When the computing system has identified the M-mode bounding box(es) (e.g., in some case using user input or in some cases automatically), the B-Mode video clip is divided into standardized segments, known as "clip segments", that are each 3 seconds in duration (though other durations may also be used), consistent with the amount of time required for clinicians to interpret for lung sliding, for example as described in Lichtenstein, "Lung ultrasound in the critically ill," Annals of intensive care, 4(1), 1-12 (2014). Clip segments are taken from the beginning of each clip, and segment overlap is permitted to ensure complete coverage. For example, if the video clip is 7 seconds in duration, three clip segments will be produced; one for 0:00-0:03, one for 0:03-0:06, and one for 0:040:07. M-modes are produced for each x-coordinate within the pleural line bounding box(es), for each clip segment.

In some cases, different approaches are used for pleural line detection, which are described below.

In a first object detection approach, a machine learning model is trained to predict the locations and sizes of bounding boxes that may contain the pleural line. In some cases, lung ultrasound experts annotate several B-mode videos with frame-level bounding boxes. The boxes may be specified in either of the following manners:
- (x, y) coordinate of top left corner, width, and height
- (x, y) coordinates of both the top left and bottom right corners

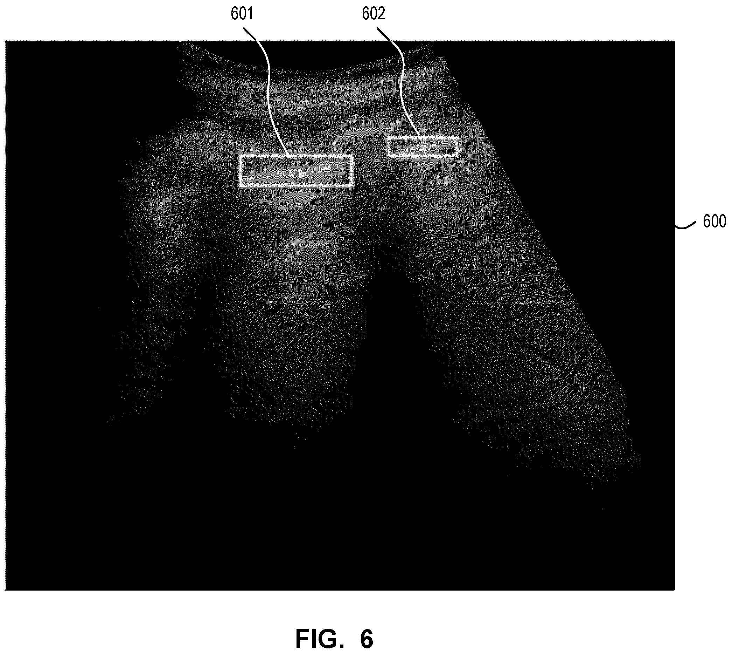

An object detection model with a standard architecture and objective function may be trained to output the location of one or more fragments of the pleural line. FIG. 6 provides an example of a B-mode image 600 where multiple pleural line fragments are separated by rib shadows. In some cases, detection architectures for image processing include, but are not limited to, the You Only Look Once (YOLO) objection detection, region-based convolutional neutral network (R-CNN) family, or single shot detector (SSD) families. Predicted bounding boxes 601 and 602 are shown around the fragments of a pleural line. Box predictions may be retained if their predicted confidence is above a threshold. Any x-coordinate within any of the predicted bounding boxes are valid locations at which an M-mode image may be taken.

In a second, explicitly programmed approach, and to avoid including a separate machine learning model in this method, the following procedure may be used to identify the single strongest pleural line fragment candidate. The values of the parameters are tuned for B-modes. The approach may be applied to either entire video clips or to 3-second clip segments.
1. Clip (segment) average: The pixel intensities are averaged across the time dimension of the B-mode video (see FIG. 5A panel ii, FIG. 5B panel ii, and FIG. 5C panel ii for examples)
2. Normalization: Rescale the clip average's pixel values such that all pixel intensities are in [0, 1].
3. Increase contrast: Multiply each pixel x by e^{k*x}, where k is set to be 4.5 for best results. This increases the contrast of the image. Renormalize by dividing by the new maximum.
4. Radon Transform: Apply the Radon Transform (see, e.g., Roulston et al., "Synthesizing radar maps of polar regions with a Doppler-only method," Applied Optics, 36(17), 3912-3919) for a range of angles in 70-110 degrees. Find the brightest point on the resulting sinogram. Use the angle of this point to rotate the image such that the pleural line, A-lines, and ribs are horizontally oriented.
5. Thresholding: Apply global thresholding with a lower bound of 40 to create a mask of where the pleural line might be. Apply adaptive thresholding with a block size of 21 and per-block mean subtraction constant of 5. Use the global thresholding mask to extract the region of interest from the adaptive threshold result.
6. Horizontal Erosion: Let w be the width of the image. Create a structuring element with shape (w//25, 1). Erode the image for 1 iteration. If this results in an image is black, restore the result of step 5.
7. Horizontal Dilation: Dilate the image with same structuring element from step 6 for 1 iteration.
8. Find contours: Apply a contour finding algorithm to retrieve the boundaries of each possible object in the image, which should include the pleural line.
9. Narrow down choices of contours: Let w and h be the width and height of the image, respectively. Eliminate contours with 0 area. Eliminate contours with an area that is less than equal to 3% of the image area and whose height is greater than or equal to a tenth of h or whose width is less than or equal to a twelfth of w. Eliminate contours with a perimeter-to-area ratio that is less than or equal to 0.3.
10. Choose best contour: Select the contour for which the sum of pixel intensities in coordinates that are below it (within x-coordinate bounds) is the greatest. If there are no contours left over from step 9, select the eliminated contour with the greatest width.
11. Double-check best contour: Identify all contours directly above or below the best contour. Among the current best contour and the newly identified contours, return the contour whose 20 brightest constituent pixel intensities have the greatest sum.

A description of a deep learning approach to M-mode classification for the absence or presence of lung sliding follows.

All M-mode images are resized to a fixed dimension and pixel intensities are rescaled to a fixed range. A convolutional neural network image binary classifier is trained to distinguish between present versus absent lung sliding. The output of the network is the confidence in absent lung sliding (p). Examples of lung point are excluded from the training and validation sets for the classifier so that a clip-wise label can be adopted, ensuring that all valid M-modes in the clip have the same label.

The output of this step is a prediction confidence for each M-mode in each 3-second clip segment. In some cases, the video clip is divided into m-second clip segments. In some cases, including the examples herein, m is 3 seconds. Other time lengths could be used to divide the video clip into segments.

A clip classification algorithm, which is executed by the clip prediction module 258, receives the prediction confidences from each M-mode in each 3-second clip segment as input, and it outputs a binary decision for "Lung Sliding Present" or "Lung Sliding Absent" for the entire clip. Generally, the algorithm will output "Absent Lung Sliding" if there is any evidence of absent lung sliding at any point of the pleural line, throughout the duration of the clip. Since it is expected that there may be noise in the M-mode confidences, multiple methods may be used for applying this clinical logic. Alternative methods are described in the subsections below.

In some cases, the below definitions regarding clip segments are used throughout these methods:
- Binning: Divide the pleural line bounding box into b horizontally spaced segments with equal width (see FIG. 5 for example)
- Brightness: Sum of pixel intensities of an M-mode image
- Threshold: Classification threshold t. If prediction confidence p ≥ t, then the prediction for an individual M-mode is "Absent Lung Sliding". If p < t, the prediction is "Lung Sliding".
- Moving average: Given a list of M-mode prediction confidences in the same clip segment ordered from left to right, obtain a smoothed set of prediction confidences by taking the moving average with window size w, where w is an integer greater than 1.

In some cases, the described methods involve the following:
1. For each 3-second clip segment:
   a. Perform binning for each clip segment, to divide the x-coordinates of the pleural line into contiguous chunks with equal width.
   b. Obtain a predicted class for each 3-second clip segment. Optionally preceded by taking a moving average of the M-mode prediction confidences for each clip segment. Clip segment predictions are obtained by checking each bin for absent lung sliding.
2. If any 3-second clip segment's prediction is "Absent Lung Sliding", then the entire clip's prediction is "Absent Lung Sliding".

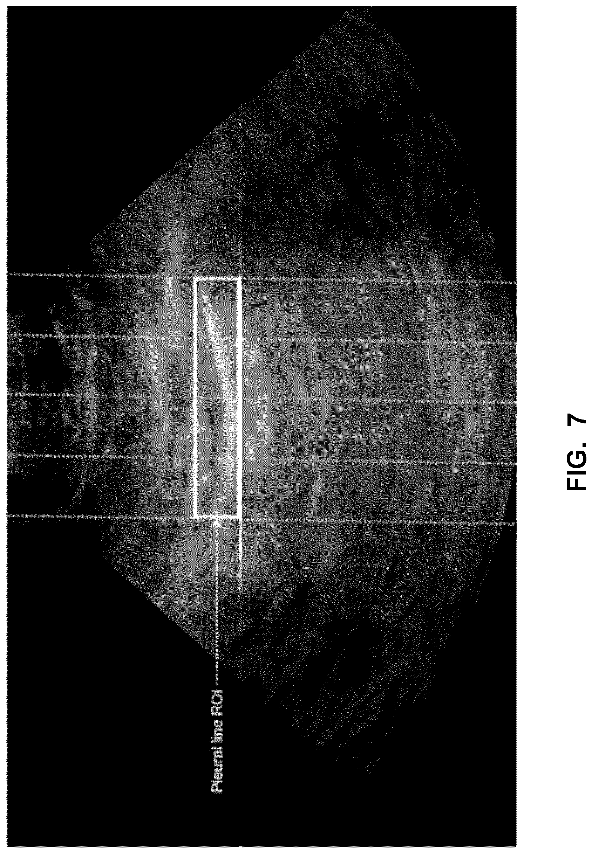

Several embodiments are described, each of which are generally similar to each other, with the exception of step 1. Below are the descriptions of four embodiments. FIG. 7 provides a visual accompaniment to understanding the process of producing a prediction for a 3-second clip segment, given its M-mode prediction confidences.

### Method 1

1. For each 3-second clip segment:
   a. Perform binning for each clip segment, to divide the x-coordinates of the pleural line into contiguous chunks with equal width.
   b. Take the brightest M-mode image in each bin.
   c. Apply the classification threshold to the prediction confidence for each selected M-mode to get a class prediction for each bin.
   d. If any of the predictions are "Absent Lung Sliding", the clip segment's prediction is set to "Absent Lung Sliding".
2. If any clip segment's prediction is "Absent Lung Sliding", then the entire clip's prediction is "Absent Lung Sliding".

### Method 2

1. For each 3-second clip segment:
   a. Perform binning for each clip segment, to divide the x-coordinates of the pleural line into contiguous chunks with equal width.
   b. For each bin, determine the mean prediction confidence for its constituent M-modes.
   c. Apply the classification threshold to the averaged prediction confidence to get a class prediction for each bin.
   d. If any of the predictions are "Absent Lung Sliding", the clip segment's prediction is set to "Absent Lung Sliding".
2. If any clip segment's prediction is "Absent Lung Sliding", then the entire clip's prediction is "Absent Lung Sliding".

### Method 3

1. For each 3-second clip segment:
   a. Perform binning for each clip segment, to divide the x-coordinates of the pleural line into contiguous chunks with equal width.
   b. Replace the list of prediction confidences for the current clip segment with its moving average.
   c. Compute a moving average (with the same window size as in b.) of the brightness of each M-mode at each x-coordinate of the pleural line.
   d. Take the M-mode image in each bin with the greatest brightness moving average.
   e. Apply the classification threshold to the prediction confidence for each selected M-mode to get a class prediction for each bin.
   f. If any of the predictions are "Absent Lung Sliding", the clip segment's prediction is set to "Absent Lung Sliding".
2. If any clip segment's prediction is "Absent Lung Sliding", then the entire clip's prediction is "Absent Lung Sliding".

### Method 4

1. For each 3-second clip segment:
   a. Perform binning for each clip segment, to divide the x-coordinates of the pleural line into contiguous chunks with equal width.
   b. Replace the list of prediction confidences for the current clip segment with its moving average.
   c. Take the M-mode corresponding to the midpoint of each bin.
   d. Apply the classification threshold to the prediction confidence for each selected M-mode to get a class prediction for each bin.
   e. If any of the predictions are "Absent Lung Sliding", the clip segment's prediction is set to "Absent Lung Sliding".
2. If any clip segment's prediction is "Absent Lung Sliding", then the entire clip's prediction is "Absent Lung Sliding".

### Method 5

1. For each 3-second clip segment:
   a. Perform binning for each clip segment, to divide the x-coordinates of the pleural line into contiguous chunks with equal width.
   b. In each bin, take the M-mode corresponding to the midpoint of the range of prediction confidences for that bin.
   c. Apply the classification threshold to the prediction confidence for each selected M-mode to get a class prediction for each bin.
   d. If any of the predictions are "Absent Lung Sliding", the clip segment's prediction is set to "Absent Lung Sliding".
2. If any clip segment's prediction is "Absent Lung Sliding", then the entire clip's prediction is "Absent Lung Sliding".

### Method 6

2. For each 3-second clip segment:
   a. Perform binning for each clip segment, to divide the x-coordinates of the pleural line into contiguous chunks with equal width.
   b. In each bin, take the M-mode corresponding to the median of the prediction confidences for that bin.
   c. Apply the classification threshold to the prediction confidence for each selected M-mode to get a class prediction for each bin.
   d. If any of the predictions are "Absent Lung Sliding", the clip segment's prediction is set to "Absent Lung Sliding".
3. If any clip segment's prediction is "Absent Lung Sliding", then the entire clip's prediction is "Absent Lung Sliding".

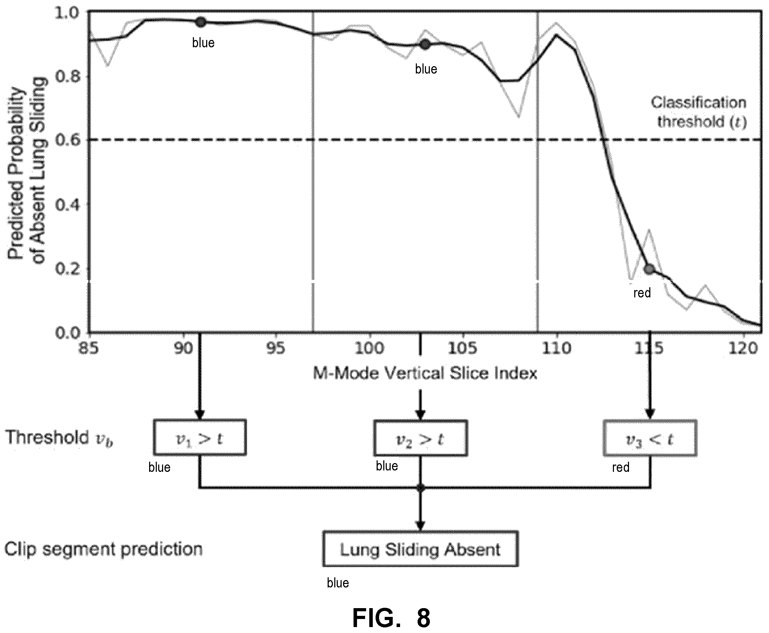

FIG. 8 provides a visual representation of step 1 for Method 4 applied to a single clip segment. For a given 3-second clip segment, the contiguous set of M-Mode-level prediction probabilities outputted by the image classifier (grey curve) are smoothed by computing a moving average with window size w (bolded black curve). The smoothed probabilities are divided into b bins and those at the midpoint of each bin (v_{b}; circular markers) are selected. If any of the selected values exceed the classification threshold (t; dashed line), then a label of "Lung Sliding Absent" (blue) is assigned to that clip segment. Otherwise, a label of "Lung Sliding Present" (red) is assigned. In this example, w=3, b=3 and t=0.6.

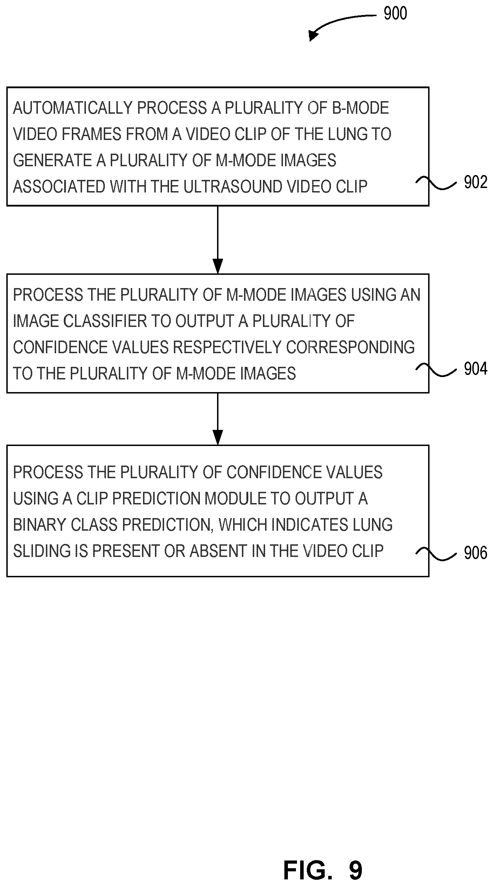

Referring to FIG. 9, in some cases, a process 900 executed by the computing system 230 includes the following operations.

Block 902: Automatically processing a plurality of B-mode video frames from a video clip of the lung to generate a plurality of M-mode images associated with the ultrasound video clip.

Block 904: Processing the plurality of M-mode images using an image classifier to output a plurality of confidence values respectively corresponding to the plurality of M-mode images; and
Block 906: Processing the plurality of confidence values using a clip prediction module to output a binary class prediction, which indicates lung sliding is present or absent in the video clip.

Various systems or processes have been described to provide examples of embodiments of the claimed subject matter. No such example embodiment described limits any claim and any claim may cover processes or systems that differ from those described. The claims are not limited to systems or processes having all the features of any one system or process described above or to features common to multiple or all the systems or processes described above. It is possible that a system or process described above is not an embodiment of any exclusive right granted by issuance of this patent application. Any subject matter described above and for which an exclusive right is not granted by issuance of this patent application may be the subject matter of another protective instrument, for example, a continuing patent application, and the applicants, inventors or owners do not intend to abandon, disclaim or dedicate to the public any such subject matter by its disclosure in this document.

For simplicity and clarity of illustration, reference numerals may be repeated among the figures to indicate corresponding or analogous elements. In addition, numerous specific details are set forth to provide a thorough understanding of the subject matter described herein. However, it will be understood by those of ordinary skill in the art that the subject matter described herein may be practiced without these specific details. In other instances, well-known methods, procedures, and components have not been described in detail so as not to obscure the subject matter described herein.

As used herein, the wording "and/or" is intended to represent an inclusive- or. That is, "X and/or Y" is intended to mean X or Y or both, for example. As a further example, "X, Y, and/or Z" is intended to mean X or Y or Z or any combination thereof.

Terms of degree such as "substantially", "about", and "approximately" as used herein mean a reasonable amount of deviation of the modified term such that the result is not significantly changed. These terms of degree may also be construed as including a deviation of the modified term if this deviation would not negate the meaning of the term it modifies.

Any recitation of numerical ranges by endpoints herein includes all numbers and fractions subsumed within that range (e.g., 1 to 5 includes 1, 1.5, 2, 2.75, 3, 3.90, 4, and 5). It is also to be understood that all numbers and fractions thereof are presumed to be modified by the term "about" which means a variation of up to a certain amount of the number to which reference is being made if the result is not significantly changed.

Some elements herein may be identified by a part number, which is composed of a base number followed by an alphabetical or subscript-numerical suffix (e.g. 112a, or 1121). All elements with a common base number may be referred to collectively or generically using the base number without a suffix (e.g. 112).

The systems and methods described herein may be implemented as a combination of hardware or software. In some cases, the systems and methods described herein may be implemented, at least in part, by using one or more computer programs, executing on one or more programmable devices including at least one processing element, and a data storage element (including volatile and non-volatile memory and/or storage elements). These systems may also have at least one input device (e.g. a pushbutton keyboard, mouse, a touchscreen, and the like), and at least one output device (e.g. a display screen, a printer, a wireless radio, and the like) depending on the nature of the device. Further, in some examples, one or more of the systems and methods described herein may be implemented in or as part of a distributed or cloud-based computing system having multiple computing components distributed across a computing network. For example, the distributed or cloud-based computing system may correspond to a private distributed or cloud-based computing cluster that is associated with an organization. Additionally, or alternatively, the distributed or cloud-based computing system be a publicly accessible, distributed or cloud-based computing cluster, such as a computing cluster maintained by Microsoft Azure^{™}, Amazon Web Services^{™}, Google Cloud^{™}, or another third-party provider.

Some elements that are used to implement at least part of the systems, methods, and devices described herein may be implemented via software that is written in a high-level procedural language such as object-oriented programming language. Accordingly, the program code may be written in any suitable programming language such as Python or Java, for example. Alternatively, or in addition thereto, some of these elements implemented via software may be written in assembly language, machine language or firmware as needed. In either case, the language may be a compiled or interpreted language.

At least some of these software programs may be stored on a storage media (e.g., a computer readable medium such as, but not limited to, read-only memory, magnetic disk, optical disc) or a device that is readable by a general or special purpose programmable device. The software program code, when read by the programmable device, configures the programmable device to operate in a new, specific, and predefined manner to perform at least one of the methods described herein.

Furthermore, at least some of the programs associated with the systems and methods described herein may be capable of being distributed in a computer program product including a computer readable medium that bears computer usable instructions for one or more processors. The medium may be provided in various forms, including non-transitory forms such as, but not limited to, one or more diskettes, compact disks, tapes, chips, and magnetic and electronic storage. Alternatively, the medium may be transitory in nature such as, but not limited to, wire-line transmissions, satellite transmissions, internet transmissions (e.g. downloads), media, digital and analog signals, and the like. The computer usable instructions may also be in various formats, including compiled and non-compiled code.

While the above description provides examples of one or more processes or systems, it will be appreciated that other processes or systems may be within the scope of the accompanying claims.

To the extent any amendments, characterizations, or other assertions previously made (in this or in any related patent applications or patents, including any parent, sibling, or child) with respect to any art, prior or otherwise, could be construed as a disclaimer of any subject matter supported by the present disclosure of this application, Applicant hereby rescinds and retracts such disclaimer. Applicant also respectfully submits that any prior art previously considered in any related patent applications or patents, including any parent, sibling, or child, may need to be re-visited.

## Claims

1. A method for processing medical imagery of a lung, the method executed in a computing environment comprising one or more processors and memory, the method comprising:
automatically processing a plurality of B-mode video frames from a video clip of the lung to generate a plurality of M-mode images associated with the video clip;
processing the plurality of M-mode images using an image classifier to output a plurality of confidence values respectively corresponding to the plurality of M-mode images; and
processing the plurality of confidence values using a clip prediction module to output a binary class prediction, which indicates lung sliding is present or absent in the video clip.

2. The method of claim 1, further comprising adding a bounding box to the plurality of B-mode video frames, wherein the bounding box encompasses a pleural line, and the plurality of M-mode images intersect the bounding box.

3. The method of claim 2, further comprising executing a machine learning model to image process one or more of the plurality of B-mode video frames to compute a location of the bounding box that encompasses the pleural line.

4. The method of claim 2, further comprising executing a process to determine a location of the bounding box, the process comprising:
computing a video clip average of pixel intensities across a time dimension of the plurality of B-mode video frames;
rescaling all pixel intensities across the plurality of B-mode video frames using the video clip average, with rescaled pixel intensities in a range [0, 1];
increasing an image contrast in each of the plurality of B-mode video frames;
applying a Radon Transform to rotate the plurality of B-mode video frames;
applying a thresholding process to extract a region of interest in the plurality of B-mode video frames;
applying horizontal erosion and horizontal dilation to the plurality of B-mode video frames;
applying a contour finding process to identify a plurality of contours that potentially bound the pleural line;
identifying a brightest contour from amongst the plurality of contours that comprises a sum of pixel intensities that is greatest, wherein the sum of pixel intensities are associated with coordinates which are below and within x-coordinate bounds of the brightest contour; and
computing the bounding box around the brightest contour.

5. The method of claim 2, further comprising dividing the video clip into a plurality of clip segments; and
for each one of the plurality of clip segments:
a. performing binning for the clip segment, to divide x-coordinates of the pleural line into contiguous chunks with equal width;
b. obtaining a predicted class for the clip segment; and
when at least one of the clip segments has the predicted class indicating an absence of lung sliding, outputting a prediction for the video clip indicating the absence of lung sliding.

6. The method of claim 5, wherein, prior to obtaining the predicted class for the clip segment, the method further comprising computing a moving average of a subset of the plurality of confidence values corresponding to the clip segment.

7. The method of claim 2, further comprising dividing the video clip into a plurality of clip segments; and
for each one of the plurality of clip segments:
a. performing binning for the clip segment, to divide x-coordinates of the pleural line into contiguous chunks with equal width, resulting in a plurality of bins;
b. identifying a brightest M-mode image in each of the plurality of bins;
c. applying a classification thresholding process to a prediction confidence for each one of the brightest M-mode images to obtain a class prediction for each of the plurality of bins;
d. when at least one of the class predictions indicates an absence of lung sliding, setting a prediction of the clip segment indicating the absence of lung sliding; and
when at least one of the plurality of clip segments has the prediction indicating the absence of lung sliding, then outputting a prediction for the video clip indicating the absence of lung sliding.

8. The method of claim 2, further comprising dividing the video clip into a plurality of clip segments; and
for each one of the plurality of clip segments:
a. performing binning for the clip segment, to divide x-coordinates of the pleural line into contiguous chunks with equal width, resulting in a plurality of bins;
b. for each of the plurality of bins, determining a mean prediction confidence for its constituent M-mode images;
c. applying a classification thresholding process to the mean prediction confidence to compute a class prediction for each of the plurality of bins;
d. when at least one of the class predictions indicates an absence of lung sliding, setting a prediction of the clip segment indicating the absence of lung sliding; and
when at least one of the plurality of clip segments has the prediction indicating the absence of lung sliding, then outputting a prediction for the video clip indicating the absence of lung sliding.

9. The method of claim 2, further comprising dividing the video clip into a plurality of clip segments; and
for each one of the plurality of clip segments:
a. performing binning for the clip segment, to divide x-coordinates of the pleural line into contiguous chunks with equal width, resulting in a plurality of bins;
b. replacing a list of prediction confidences for the clip segment with its moving average;
c. computing a moving average of a brightness of each M-mode image at each x-coordinate of the pleural line;
d. identifying a M-mode image in each of the plurality of bins with the greatest brightness moving average;
e. applying a classification thresholding process to a prediction confidence for each identified M-mode image to compute a class prediction for each of the plurality of bins;
f. when at least one of the class predictions indicate an absence of lung sliding, setting a prediction of the clip segment indicating the absence of lung sliding; and
when at least one of the plurality of clip segments has the prediction indicating the absence of lung sliding, then outputting a prediction for the video clip indicating the absence of lung sliding.

10. The method of claim 2, further comprising dividing the video clip into a plurality of clip segments; and
for each one of the plurality of clip segments:
a. performing binning for the clip segment, to divide x-coordinates of the pleural line into contiguous chunks with equal width, resulting in a plurality of bins;
b. replacing a list of prediction confidences for the clip segment with its moving average;
c. identifying a M-mode image corresponding to a midpoint of each bin from the plurality of bins;
d. applying a classification thresholding process to a prediction confidence for each identified M-mode image to compute a class prediction for each of the plurality of bins;
e. when at least one of the class predictions indicate an absence of lung sliding, setting a prediction of the clip segment indicating the absence of lung sliding; and
when at least one of the plurality of clip segments has the prediction indicating the absence of lung sliding, then outputting a prediction for the video clip indicating the absence of lung sliding.

11. The method of claim 2, further comprising dividing the video clip into a plurality of clip segments; and
for each one of the plurality of clip segments:
a. performing binning for the clip segment, to divide x-coordinates of the pleural line into contiguous chunks with equal width, resulting in a plurality of bins;
b. identifying a M-mode image corresponding to a midpoint of a range of prediction confidences for each bin from amongst the plurality of bins;
c. applying a classification thresholding process to a prediction confidence for each identified M-mode image to compute a class prediction for each of the plurality of bins;
d. when at least one of the class predictions indicates an absence of lung sliding, setting a prediction of the clip segment indicating the absence of lung sliding; and
when at least one of the plurality of clip segments has the prediction indicating the absence of lung sliding, then outputting a prediction for the video clip indicating the absence of lung sliding.

12. The method of claim 2, further comprising dividing the video clip into a plurality of clip segments; and
for each one of the plurality of clip segments:
a. performing binning for the clip segment, to divide x-coordinates of the pleural line into contiguous chunks with equal width, resulting in a plurality of bins;
b. for each one of the plurality of bins, identifying a M-mode image corresponding to a median of prediction confidences for that bin;
c. applying a classification thresholding process to a prediction confidence for each identified M-mode image to compute a class prediction for each of the plurality of bins;
d. when at least one of the class predictions indicates an absence of lung sliding, setting a prediction of the clip segment indicating the absence of lung sliding; and
when at least one of the plurality of clip segments has the prediction indicating the absence of lung sliding, then outputting a prediction for the video clip indicating the absence of lung sliding.

13. A computing system for processing medical imagery of a lung, comprising:
a memory storing instructions; and
a processor coupled to the memory, the processor being configured to execute the instructions to perform the method of any preceding claim.

## Patentansprüche

1. Verfahren zur Verarbeitung medizinischer Bilddaten einer Lunge, wobei das Verfahren in einer Computerumgebung mit einem oder mehreren Prozessoren und einem Speicher ausgeführt wird und Folgendes umfasst:
automatisches Verarbeiten mehrerer B-Modus-Videobilder aus einem Videoclip der Lunge zur Erzeugung mehrerer M-Modus-Bilder, die dem Videoclip zugeordnet sind;
Verarbeiten der Vielzahl von M-Modus-Bildern mithilfe eines Bildklassifikators zur Ausgabe einer Vielzahl von Konfidenzwerten, die jeweils der Vielzahl von M-Modus-Bildern entsprechen; und
Verarbeiten der Vielzahl von Konfidenzwerten mithilfe eines Clip-Vorhersagemoduls, um eine binäre Klassenvorhersage auszugeben, die angibt, ob im Videoclip ein Gleiten der Lunge vorhanden ist oder nicht.

2. Verfahren nach Anspruch 1, ferner umfassend das Hinzufügen eines Begrenzungsrahmens zu der Vielzahl von B-Modus-Videobildern, wobei der Begrenzungsrahmen eine Pleura-Linie umschließt und die Vielzahl von M-Modus-Bildern den Begrenzungsrahmen schneidet.

3. Verfahren nach Anspruch 2, ferner umfassend das Ausführen eines Modells für maschinelles Lernen zur Bildverarbeitung eines oder mehrerer der mehreren B-Modus-Videobilder, um die Position des Begrenzungsrahmens zu berechnen, der die Pleura-Linie umschließt.

4. Verfahren nach Anspruch 2, ferner umfassend das Durchführen eines Prozesses zur Bestimmung der Position des Begrenzungsrahmens, wobei der Prozess Folgendes umfasst:
Berechnen eines Videoclip-Mittelwerts der Pixelintensitäten über eine Zeitdimension der Vielzahl von B-Modus-Videobildern;
Neuskalieren der Intensitäten aller Pixel in den B-Modus-Videobildern mithilfe des Mittelwerts des Videoclips, wobei die neu skalierten Pixelintensitäten in einem Bereich von [0, 1] liegen;
Erhöhen des Bildkontrasts in jedem der mehreren B-Modus-Videobilder;
Anwenden einer Radon-Transformation zur Rotation der mehreren B-Modus-Videobildern;
Anwenden eines Schwellenwertverfahrens zur Extraktion eines relevanten Bereichs aus der Vielzahl von B-Modus-Videobildern;
Anwenden horizontaler Erosion und horizontaler Dilatation auf die Vielzahl von B-Modus-Videobildern;
Anwenden eines Konturdetektionsverfahrens zur Identifizierung einer Vielzahl von Konturen, die möglicherweise die Pleura-Linie begrenzen;
Identifizieren der hellsten Kontur aus einer Vielzahl von Konturen, die die größte Summe der Pixelintensitäten aufweist, wobei die Summe der Pixelintensitäten Koordinaten zugeordnet ist, die unterhalb und innerhalb der x-Koordinatengrenzen der hellsten Kontur liegen; und
Berechnen des Begrenzungsrahmens um die hellste Kontur.

5. Verfahren nach Anspruch 2, ferner umfassend das Aufteilen des Videoclips in eine Vielzahl von Clipsegmenten; und
für jedes der mehreren Clipsegmente:
a. Durchführen des Binning für das Clip-Segment, um die x-Koordinaten der Pleura-Linie in zusammenhängende Abschnitte gleicher Breite zu unterteilen;
b. Ermitteln einer vorhergesagten Klasse für das Clipsegment; und
wenn mindestens eines der Clipsegmente die vorhergesagte Klasse aufweist, die auf das Fehlen eines Lungengleitens hinweist, Ausgeben einer Vorhersage für den Videoclip, die auf das Fehlen eines Lungengleitens hinweist.

6. Verfahren nach Anspruch 5, wobei vor dem Ermitteln der vorhergesagten Klasse für das Clipsegment das Verfahren ferner das Berechnen eines gleitenden Durchschnitts einer Teilmenge der Vielzahl von Konfidenzwerten umfasst, die dem Clipsegment entsprechen.

7. Verfahren nach Anspruch 2, ferner umfassend das Aufteilen des Videoclips in eine Vielzahl von Clipsegmenten; und
für jedes der mehreren Clipsegmente:
a. Durchführen einer Binning-Operation für das Clip-Segment, um die x-Koordinaten der Pleura-Linie in zusammenhängende Abschnitte gleicher Breite zu unterteilen, wodurch eine Vielzahl von Bins entsteht;
b. Identifizieren des hellsten M-Modus-Bildes in jedem der mehreren Bins;
c. Anwenden eines Klassifizierungsschwellenwertverfahrens auf die Vorhersagekonfidenz jedes der hellsten M-Modus-Bilder, um eine Klassenvorhersage für jedes der mehreren Bins zu erhalten;
d. wenn mindestens eine der Klassenvorhersagen auf das Fehlen eines Lungengleitens hinweist, Festlegen einer Vorhersage für das Clipsegment, die das Fehlen eines Lungengleitens anzeigt; und
wenn mindestens eines der Segmente des Videoclips die Vorhersage enthält, dass kein Lungengleiten stattfindet, Ausgeben einer Vorhersage für den Videoclip, die das Fehlen eines Lungengleitens anzeigt.

8. Verfahren nach Anspruch 2, ferner umfassend das Aufteilen des Videoclips in eine Vielzahl von Clipsegmenten; und
für jedes der mehreren Clipsegmente:
a. Durchführen einer Binning-Operation für das Clip-Segment, um die x-Koordinaten der Pleura-Linie in zusammenhängende Abschnitte gleicher Breite zu unterteilen, wodurch eine Vielzahl von Bins entsteht;
b. für jedes der mehreren Bins, Bestimmen einer mittleren Vorhersagekonfidenz für die darin enthaltenen M-Modus-Bilder;
c. Anwenden eines Klassifizierungsschwellenwertverfahrens auf die mittlere Vorhersagekonfidenz, um für jedes der mehreren Bins eine Klassenvorhersage zu berechnen;
d. wenn mindestens eine der Klassenvorhersagen auf das Fehlen eines Lungengleitens hinweist, Festlegen einer Vorhersage für das Clipsegment, die das Fehlen eines Lungengleitens anzeigt; und
wenn mindestens eines der Segmente des Videoclips die Vorhersage enthält, dass kein Lungengleiten stattfindet, Ausgeben einer Vorhersage für den Videoclip, die das Fehlen eines Lungengleitens anzeigt.

9. Verfahren nach Anspruch 2, ferner umfassend das Aufteilen des Videoclips in eine Vielzahl von Clipsegmenten; und
für jedes der mehreren Clipsegmente:
a. Durchführen einer Binning-Operation für das Clip-Segment, um die x-Koordinaten der Pleura-Linie in zusammenhängende Abschnitte gleicher Breite zu unterteilen, wodurch eine Vielzahl von Bins entsteht;
b. Ersetzen einer Liste von Vorhersagekonfidenzen für das Clipsegment durch seinen gleitenden Durchschnitt;
c. Berechnen eines gleitenden Mittelwerts der Helligkeit jedes M-Modus-Bildes an jeder x-Koordinate der Pleura-Linie;
d. Identifizieren eines M-Modus-Bildes in jedem der mehreren Bins mit dem größten gleitenden Mittelwert der Helligkeit;
e. Anwenden eines Klassifizierungsschwellenwertverfahrens auf die Vorhersagekonfidenz jedes identifizierten M-Modus-Bildes, um eine Klassenvorhersage für jedes der mehreren Bins zu berechnen;
f. wenn mindestens eine der Klassenvorhersagen auf das Fehlen eines Lungengleitens hinweist, Festlegen einer Vorhersage für das Clipsegment, die das Fehlen eines Lungengleitens anzeigt; und
wenn mindestens eines der mehreren Segmente des Videoclips die Vorhersage enthält, dass kein Lungengleiten stattfindet, Ausgeben einer Vorhersage für den Videoclip, die das Fehlen eines Lungengleitens anzeigt.

10. Verfahren nach Anspruch 2, ferner umfassend das Aufteilen des Videoclips in eine Vielzahl von Clipsegmenten; und
für jedes der mehreren Clipsegmente:
a. Durchführen einer Binning-Operation für das Clip-Segment, um die x-Koordinaten der Pleura-Linie in zusammenhängende Abschnitte gleicher Breite zu unterteilen, wodurch eine Vielzahl von Bins entsteht;
b. Ersetzen einer Liste von Vorhersagekonfidenzen für das Clipsegment durch seinen gleitenden Durchschnitt;
c. Identifizieren eines M-Modus-Bildes, das dem Mittelpunkt jedes Bins aus der Vielzahl der Bins entspricht;
d. Anwenden eines Klassifizierungsschwellenwertverfahrens auf die Vorhersagekonfidenz jedes identifizierten M-Modus-Bildes, um eine Klassenvorhersage für jedes der mehreren Bins zu berechnen;
e. wenn mindestens eine der Klassenvorhersagen auf das Fehlen eines Lungengleitens hinweist, Festlegen einer Vorhersage für das Clipsegment, die das Fehlen eines Lungengleitens anzeigt; und
wenn mindestens eines der mehreren Segmente des Videoclips die Vorhersage enthält, dass kein Lungengleiten stattfindet, Ausgeben einer Vorhersage für den Videoclip, die das Fehlen eines Lungengleitens anzeigt.

11. Verfahren nach Anspruch 2, ferner umfassend das Aufteilen des Videoclips in eine Vielzahl von Clipsegmenten; und
für jedes der mehreren Clipsegmente:
a. Durchführen einer Binning-Operation für das Clip-Segment, um die x-Koordinaten der Pleura-Linie in zusammenhängende Abschnitte gleicher Breite zu unterteilen, wodurch eine Vielzahl von Bins entsteht;
b. Identifizieren eines M-Modus-Bildes, das dem Mittelpunkt eines Bereichs von Vorhersagekonfidenzen für jedes Bin aus der Vielzahl der Bins entspricht;
c. Anwenden eines Klassifizierungsschwellenwertverfahrens auf die Vorhersagekonfidenz jedes identifizierten M-Modus-Bildes, um eine Klassenvorhersage für jedes der mehreren Bins zu berechnen;
d. wenn mindestens eine der Klassenvorhersagen auf das Fehlen eines Lungengleitens hinweist, Festlegen einer Vorhersage für das Clipsegment, die das Fehlen eines Lungengleitens anzeigt; und
wenn mindestens eines der mehreren Segmente des Videoclips die Vorhersage enthält, dass kein Lungengleiten stattfindet, Ausgeben einer Vorhersage für den Videoclip, die das Fehlen eines Lungengleitens anzeigt.

12. Verfahren nach Anspruch 2, ferner umfassend das Aufteilen des Videoclips in eine Vielzahl von Clipsegmenten; und
für jedes der mehreren Clipsegmente:
a. Durchführen einer Binning-Operation für das Clip-Segment, um die x-Koordinaten der Pleura-Linie in zusammenhängende Abschnitte gleicher Breite zu unterteilen, wodurch eine Vielzahl von Bins entsteht;
b. für jedes der mehreren Bins, Identifizieren eines M-Modus-Bildes, das dem Median der Vorhersagekonfidenzen für dieses Bin entspricht;
c. Anwenden eines Klassifizierungsschwellenwertverfahrens auf die Vorhersagekonfidenz jedes identifizierten M-Modus-Bildes, um eine Klassenvorhersage für jedes der mehreren Bins zu berechnen;
d. wenn mindestens eine der Klassenvorhersagen auf das Fehlen eines Lungengleitens hinweist, Festlegen einer Vorhersage für das Clipsegment, die das Fehlen eines Lungengleitens anzeigt; und
wenn mindestens eines der Segmente des Videoclips die Vorhersage enthält, dass kein Lungengleiten stattfindet, Ausgeben einer Vorhersage für den Videoclip, die das Fehlen eines Lungengleitens anzeigt.

13. Computersystem zum Verarbeiten medizinischer Bilddaten einer Lunge, umfassend:
einen Datenspeicher, der Anweisungen speichert; und
einen mit dem Speicher gekoppelten Prozessor, wobei der Prozessor so konfiguriert ist, dass er die Anweisungen zur Durchführung des Verfahrens nach einem der vorhergehenden Ansprüche ausführt.

## Revendications

1. Procédé de traitement d'images médicales d'un poumon, le procédé étant exécuté dans un environnement informatique comprenant un ou plusieurs processeurs et une mémoire, le procédé comprenant :
le traitement automatique d'une pluralité de trames vidéo en mode B à partir d'un clip vidéo du poumon pour générer une pluralité d'images en mode M associées au clip vidéo ;
le traitement de la pluralité d'images en mode M à l'aide d'un classificateur d'images pour produire une pluralité de valeurs de confiance correspondant respectivement à la pluralité d'images en mode M ; et
le traitement de la pluralité de valeurs de confiance à l'aide d'un module de prédiction de clip pour produire une prédiction de classe binaire, qui indique si le glissement pulmonaire est présent ou absent dans le clip vidéo.

2. Procédé selon la revendication 1, comprenant en outre l'ajout d'un cadre de délimitation à la pluralité de trames vidéo en mode B, dans lequel le cadre de délimitation englobe une ligne pleurale, et la pluralité d'images en mode M croisent le cadre de délimitation.

3. Procédé selon la revendication 2, comprenant en outre l'exécution d'un modèle d'apprentissage automatique pour traiter l'image d'une ou plusieurs de la pluralité de trames vidéo en mode B afin de calculer l'emplacement du cadre de délimitation qui englobe la ligne pleurale.

4. Procédé selon la revendication 2, comprenant en outre l'exécution d'un processus visant à déterminer un emplacement du cadre de délimitation, le processus comprenant :
le calcul d'une moyenne de clip vidéo d'intensités de pixels sur une dimension temporelle de la pluralité de trames vidéo en mode B ;
le rééchelonnement de toutes les intensités de pixels sur la pluralité de trames vidéo en mode B à l'aide de la moyenne de clip vidéo, avec des intensités de pixels rééchelonnées dans une plage de [0, 1] ;
l'augmentation d'un contraste d'image dans chacune de la pluralité de trames vidéo en mode B ;
l'application d'une transformation de Radon pour faire pivoter la pluralité de trames vidéo en mode B ;
l'application d'un processus de seuillage pour extraire une région d'intérêt dans la pluralité de trames vidéo en mode B ;
l'application de l'érosion horizontale et de la dilatation horizontale à la pluralité de trames vidéo en mode B ;
l'application d'un processus de recherche de contours pour identifier une pluralité de contours qui délimitent potentiellement la ligne pleurale ;
l'identification, parmi la pluralité de contours, d'un contour le plus lumineux qui comprend une somme d'intensités de pixels la plus élevée, dans lequel la somme d'intensité de pixels est associée à des coordonnées qui sont situées en dessous et à l'intérieur des limites de la coordonnée x du contour le plus lumineux ; et
le calcul du cadre de délimitation autour du contour le plus lumineux.

5. Procédé selon la revendication 2, comprenant en outre la division du clip vidéo en une pluralité de segments de clip ; et
pour chacun de la pluralité de segments de clip :
a. l'exécution d'un regroupement pour le segment de clip, afin de diviser les coordonnées x de la ligne pleurale en morceaux contigus de largeur égale ;
b. l'obtention d'une classe prédite pour le segment de clip ; et
lorsqu'au moins un des segments de clip présente la classe prédite indiquant une absence de glissement pulmonaire, la production d'une prédiction pour le clip vidéo indiquant l'absence de glissement pulmonaire.

6. Procédé selon la revendication 5, dans lequel, avant d'obtenir la classe prédite pour le segment de clip, le procédé comprend en outre le calcul d'une moyenne mobile d'un sous-ensemble de la pluralité de valeurs de confiance correspondant au segment de clip.

7. Procédé selon la revendication 2, comprenant en outre la division du clip vidéo en une pluralité de segments de clip ; et
pour chacun de la pluralité de segments de clip :
a. l'exécution d'un regroupement pour le segment de clip, afin de diviser les coordonnées x de la ligne pleurale en morceaux contigus de largeur égale, résultant en une pluralité de compartiments ;
b. l'identification d'une image en mode M la plus lumineuse dans chacun de la pluralité de compartiments ;
c. l'application d'un processus de seuillage de classification à une confiance de prédiction pour chacune des images en mode M les plus lumineuses afin d'obtenir une prédiction de classe pour chacun de la pluralité de compartiments ;
d. lorsqu'au moins une des prédictions de classe indique une absence de glissement pulmonaire, l'établissement d'une prédiction du segment de clip indiquant l'absence de glissement pulmonaire ; et
lorsqu'au moins un de la pluralité de segments de clip présente la prédiction indiquant l'absence de glissement pulmonaire, la production d'une prédiction pour le clip vidéo indiquant l'absence de glissement pulmonaire.

8. Procédé selon la revendication 2, comprenant en outre la division du clip vidéo en une pluralité de segments de clip ; et
pour chacun de la pluralité de segments de clip :
a. l'exécution d'un regroupement pour le segment de clip, afin de diviser les coordonnées x de la ligne pleurale en morceaux contigus de largeur égale, résultant en une pluralité de compartiments ;
b. pour chacun de la pluralité de compartiments, la détermination d'une confiance de prédiction moyenne pour ses images en mode M constitutives ;
c. l'application d'un processus de seuillage de classification à la confiance de prédiction moyenne pour calculer une prédiction de classe pour chacun de la pluralité de compartiments ;
d. lorsqu'au moins une des prédictions de classe indique une absence de glissement pulmonaire, l'établissement d'une prédiction du segment de clip indiquant l'absence de glissement pulmonaire ; et
lorsqu'au moins un de la pluralité de segments de clip présente la prédiction indiquant l'absence de glissement pulmonaire, la production d'une prédiction pour le clip vidéo indiquant l'absence de glissement pulmonaire.

9. Procédé selon la revendication 2, comprenant en outre la division du clip vidéo en une pluralité de segments de clip ; et
pour chacun de la pluralité de segments de clip :
a. l'exécution d'un regroupement pour le segment de clip, afin de diviser les coordonnées x de la ligne pleurale en morceaux contigus de largeur égale, résultant en une pluralité de compartiments ;
b. le remplacement d'une liste de confiances de prédiction pour le segment de clip par sa moyenne mobile ;
c. le calcul d'une moyenne mobile d'une luminosité de chaque image en mode M à chaque coordonnée x de la ligne pleurale ;
d. l'identification d'une image en mode M dans chacun de la pluralité de compartiments avec la moyenne mobile de luminosité la plus élevée ;
e. l'application d'un processus de seuillage de classification à une confiance de prédiction pour chaque image en mode M identifiée afin de calculer une prédiction de classe pour chacun de la pluralité de compartiments ;
f. lorsqu'au moins une des prédictions de classe indique une absence de glissement pulmonaire, l'établissement d'une prédiction du segment de clip indiquant l'absence de glissement pulmonaire ; et
lorsqu'au moins un de la pluralité de segments de clip présente la prédiction indiquant l'absence de glissement pulmonaire, la production d'une prédiction pour le clip vidéo indiquant l'absence de glissement pulmonaire.

10. Procédé selon la revendication 2, comprenant en outre la division du clip vidéo en une pluralité de segments de clip ; et
pour chacun de la pluralité de segments de clip :
a. l'exécution d'un regroupement pour le segment de clip, afin de diviser les coordonnées x de la ligne pleurale en morceaux contigus de largeur égale, résultant en une pluralité de compartiments ;
b. le remplacement d'une liste de confiances de prédiction pour le segment de clip par sa moyenne mobile ;
c. l'identification d'une image en mode M correspondant à un point médian de chaque compartiment parmi la pluralité de compartiments ;
d. l'application d'un processus de seuillage de classification à une confiance de prédiction pour chaque image en mode M identifiée afin de calculer une prédiction de classe pour chacun de la pluralité de compartiments ;
e. lorsqu'au moins une des prédictions de classe indique une absence de glissement pulmonaire, l'établissement d'une prédiction du segment de clip indiquant l'absence de glissement pulmonaire ; et
lorsqu'au moins un de la pluralité de segments de clip présente la prédiction indiquant l'absence de glissement pulmonaire, la production d'une prédiction pour le clip vidéo indiquant l'absence de glissement pulmonaire.

11. Procédé selon la revendication 2, comprenant en outre la division du clip vidéo en une pluralité de segments de clip ; et
pour chacun de la pluralité de segments de clip :
a. l'exécution d'un regroupement pour le segment de clip, afin de diviser les coordonnées x de la ligne pleurale en morceaux contigus de largeur égale, résultant en une pluralité de compartiments ;
b. l'identification d'une image en mode M correspondant à un point médian d'une plage de confiances de prédiction pour chaque compartiment parmi la pluralité de compartiments ;
c. l'application d'un processus de seuillage de classification à une confiance de prédiction pour chaque image en mode M identifiée afin de calculer une prédiction de classe pour chacun de la pluralité de compartiments ;
d. lorsqu'au moins une des prédictions de classe indique une absence de glissement pulmonaire, l'établissement d'une prédiction du segment de clip indiquant l'absence de glissement pulmonaire ; et
lorsqu'au moins un de la pluralité de segments de clip présente la prédiction indiquant l'absence de glissement pulmonaire, la production d'une prédiction pour le clip vidéo indiquant l'absence de glissement pulmonaire.

12. Procédé selon la revendication 2, comprenant en outre la division du clip vidéo en une pluralité de segments de clip ; et
pour chacun de la pluralité de segments de clip :
a. l'exécution d'un regroupement pour le segment de clip, afin de diviser les coordonnées x de la ligne pleurale en morceaux contigus de largeur égale, résultant en une pluralité de compartiments ;
b. pour chacun de la pluralité de compartiments, l'identification d'une image en mode M correspondant à une médiane de confiances de prédiction pour ce compartiment ;
c. l'application d'un processus de seuillage de classification à une confiance de prédiction pour chaque image en mode M identifiée afin de calculer une prédiction de classe pour chacun de la pluralité de compartiments ;
d. lorsqu'au moins une des prédictions de classe indique une absence de glissement pulmonaire, l'établissement d'une prédiction du segment de clip indiquant l'absence de glissement pulmonaire ; et
lorsqu'au moins un de la pluralité de segments de clip présente la prédiction indiquant l'absence de glissement pulmonaire, la production d'une prédiction pour le clip vidéo indiquant l'absence de glissement pulmonaire.

13. Système informatique pour le traitement d'images médicales d'un poumon, comprenant :
une mémoire stockant des instructions ; et
un processeur couplé à la mémoire, le processeur étant configuré pour exécuter les instructions permettant de réaliser le procédé selon une quelconque revendication précédente.
